# EUROPÄISCHE PATENTSCHRIFT

(11) **EP 1 001 993 B1**
(45) Veröffentlichungstag und Bekanntmachung des Hinweises auf die Patenterteilung: **05.12.2001**
(21) Anmeldenummer: 98946298.1
(22) Anmeldetag: 07.08.1998
(51) Int. Cl.: C08B 30/12, C08B 31/12, A61K 31/715, A61K 31/718

(54) **VERFAHREN ZUR KONTINUIERLICHEN HERSTELLUNG VON HYDROLYTISCH ABGEBAUTER GGFLS. SUBSTITUIERTER STÄRKE, VERWENDUNG DER HYDROLYTISCH ABGEBAUTEN STÄRKE UND VORRICHTUNG ZU IHRER HERSTELLUNG**
METHOD FOR THE CONTINUOUS PRODUCTION OF HYDROLYTICALLY BROKEN DOWN AND POSSIBLY SUBSTITUTED STARCH, USE OF HYDROLYTICALLY BROKEN DOWN STARCH AND DEVICE FOR PRODUCING SAME
PROCEDE DE FABRICATION EN CONTINU D'AMIDON HYDROLYTIQUEMENT DEGRADABLE EVENTUELLEMENT SUBSTITUE, UTILISATION D'AMIDON HYDROLYTIQUEMENT DEGRADE ET DISPOSITIF PERMETTANT DE PRODUIRE LEDIT AMIDON

(30) Priorität: 08.08.1997 DE 19734370; 08.10.1997 DE 19744353
(43) Veröffentlichungstag der Anmeldung: 24.05.2000
(73) Patentinhaber: Fresenius AG, 61350 Bad Homburg (DE)
(72) Erfinder: SOMMERMEYER, Klaus, D-61191 Rosbach (DE); HENNING, Klaus, D-61250 Usingen (DE); GÖRG, Michael, D-61197 Florstadt (DE); MAUL, Thomas, D-61169 Friedberg-Ockstadt (DE)
(74) Vertreter: Luderschmidt, Schüler & Partner GbR
(86) Internationale Anmeldenummer: EP9805011
(87) Internationale Veröffentlichungsnummer: WO9907743

(56) Entgegenhaltungen:
- DE-A- 2 837 067
- DE-A- 2 930 614
- DE-A- 3 604 415

## Beschreibung

Verfahren zur kontinuierlichen Herstellung von hydrolytisch abgebauter Stärke bzw. hydrolytisch abgebauten substituierten Stärkeprodukten.

Die Erfindung betrifft ein Verfahren zur Herstellung hydrolytisch abgebauter Stärke bzw. hydrolytisch abgebauter substituierter Stärkeprodukte, wie Hydroxyethylstärke oder Hydroxypropylstärke, die Verwendung der gemäß der Erfindung hergestellten Produkte im medizinischen Bereich, insbesondere als Plasmastreckmittel, sowie eine Vorrichtung zur Herstellung von hydrolytisch abgebauter Stärke bzw. hydrolytisch abgebauten substituierten Stärkeprodukten.

Es ist bekannt, Stärke und substituierte Stärkeprodukte, wie Hydroxyethyl- oder Hydroxypropylstärke, hydrolytisch abzubauen. So wird in der
DE-OS 30 000 465 ein Verfahren zur Herstellung eines Stärkehydrolysats beschrieben, bei dem α-Amylase verwendet wird. Das dort beschriebene Verfahren arbeitet sehr kompliziert und ist nicht ohne weiteres kontinuierlich durchzuführen. Es ist im übrigen nur beschränkt anwendbar.

Auch in der DE-A1-33 13 600 wird ein Verfahren zum hydrolytischen Abbau von Stärke beschrieben, bei dem α-Amylase, beta-Amylase oder Pullulanase eingesetzt wird, wobei die Stärke vor oder nach der Hydrolyse zum Beispiel mit Ethylenoxid substituiert werden kann.

Der Abbau von Hydroxyethylstärke zu einem Produkt, das als Plasma-Expander eingesetzt werden kann, wird u.a. in der EP-A1-0 402 724 beschrieben.

Die Überführung derartiger Verfahren in den kontinuierlichen Maßstab ist nicht ohne weiteres möglich. Es besteht somit ein Bedürfnis nach einem kontinuierlichen Verfahren, das wirtschaftlich arbeitet und das zu Produkten führt, die auf den verschiedensten Gebieten eingesetzt werden können.

Bekanntlich werden gerade an die Produkte, welche auf medizinischem Gebiet verwendet werden, hohe Anforderungen gestellt. Zum einen benötigt man Produkte, die beim Patienten keine Allergie hervorrufen, zum anderen soll die Abbaurate, d.h. der Konzentrationsabfall innerhalb der ersten 24 Stunden, beim Patienten sehr hoch sein und die Organhalbwertszeit kurz sein. Im übrigen hängt die klinische Verwendbarkeit von abgebauten Stärkeprodukten sehr stark von den physikalisch chemischen Eigenschaften ab. In diesem Zusammenhang wird auf die Arbeiten von Klaus Sommermeyer et al. verwiesen, die u.a. in Krankenhauspharmacie 8, (8271/8) (1987) und starch/Stärke 44 (5), 173-9(1992) erschienen sind.

Aufgabe der Erfindung ist es, ein Verfahren zur kontinuierlichen Herstellung von hydrolytisch abgebauter Stärke bzw. hydrolytisch abgebauter substituierter Stärkeprodukte zur Verfügung zu stellen, das wirtschaftlich arbeitet, mit dem die Eigenschaften der Abbauprodukte gezielt eingestellt werden können, das sich mit geringem apparativen und verfahrenstechnischen Aufbau durchführen läßt und das zu Produkten führt, die insbesondere im medizinischen Bereich und in der Lebensmitteltechnologie eingesetzt werden können.

Diese Aufgabe wird durch ein Verfahren gemäß Patentanspruch 1 gelöst. In den Patentansprüchen 2 bis 9 werden weitere vorteilhafte Ausführungsformen des erfindungsgemäßen Verfahrens beschrieben.

Ein weiterer Gegenstand der Erfindung ist die Verwendung der nach einem Verfahren gemäß den Ansprüchen 1 bis 9 hergestellten Produkte als Plasmastreckmittel bzw. zur Herstellung von Dialyselösungen. Ein weiterer Gegenstand der Erfindung ist eine Vorrichtung zur Durchführung eines Verfahrens zur kontinuierlichen Herstellung von hydrolytisch abgebauten Stärkederivaten gemäß den Ansprüchen 11 bis 13.

Zur Durchführung des erfindungsgemäßen Verfahrens können übliche Stärken eingesetzt werden, wie beispielsweise Kartoffelstärke, Weizenstärke, Maniocstärke. Besonders geeignet sind die an Amylopectin reichen Stärken, wie die wachsartige Milo (SORGHUM)-Stärke, Maisstärke oder Reis-Stärke. Die Stärken können modifiziert oder nicht modifiziert eingesetzt werden; die Stärke kann auch bereits als teilabgebaute Stärke zum Einsatz gelangen. Als modfizierte Stärken werden insbesondere Hydroxypropyl- und bevorzugt Hydroxyethylstärke eingesetzt.

Die Modifizierung kann. vor der Hydrolyse, jedoch auch nach der Hydrolyse durchgeführt werden. Bevorzugt wird jedoch vor der Hydrolyse modifiziert, insbesondere ethoxyliert.

Die abzubauende modifizierte oder nicht-modifizierte Stärke wird vorteilhafterweise als wässrige Lösung oder Suspension eingesetzt, wobei unter Suspension auch in Wasser befindliche, Stärke enthaltende Körner zu verstehen sind. Die Konzentration an Stärke oder modifizierter Stärke in der Lösung bzw. der Suspension kann in weiten Grenzen eingestellt werden.

Man kann die Konzentration bereits vor der Hydrolyse im Hinblick auf den gewünschten Verwendungszweck des Endprodukts einstellen; ferner ist es möglich, durch die Wahl der Konzentration in Zusammenspiel mit weiteren Parametern, wie Hydrolysetemperatur, Verweilzeiten, das Eigenschaftsprofil des Endprodukts zu beeinflussen. Vorzugsweise beträgt die Konzentration 25-30 Gew.-% bezogen auf das Gesamtgewicht.

Nach Versetzen mit einem Hydrolysierungsmittel, insbesondere einer Mineralsäure, wie Salzsäure, wird auf die gewünschte Temperatur erhitzt oder gekühlt, vorzugsweise mit Hilfe von Wärmeaustauschern.

Die Suspension bzw. Lösung wird sodann einem rohrförmigen temperierten Reaktor zugeleitet, wobei unter Reaktor auch eine Vielzahl von Reaktoreinheiten zu verstehen ist, die nacheinander geschaltet sind. Der Reaktor ist auf die gewünschte Hydrolysiertemperatur temperiert, vorzugsweise auf 70-80°C. Das zu hydrolysierende Gut wird dem rohrförmigen Reaktor von unten, d.h. gegen die Schwerkraft, zugeführt, so daß sich die Suspension bzw. Lösung von unten nach oben, d.h. in aufsteigender Richtung, bewegt. Sofern mehrere Reaktoreinheiten eingesetzt werden, sind sämtliche Reaktoreinheiten parallel ausgerichtet und werden jeweils von unten her angeströmt.

Grundsätzlich ist es möglich, den überwiegenden Teil der Hydrolyse in einem einzigen Rohr durchzuführen. Man kann jedoch auch mehrere rohrförmige Reaktoreinheiten aneinanderreihen, beispielsweise nebeneinander oder übereinander anordnen, was aus Herstellungs- und Handhabungsgründen bevorzugt ist. Das Überleiten des Hydrolyseguts von einer Reaktoreinheit in die nächste Reaktoreinheit kann in einfacher Weise z.B. mit Rohrverbindungen, ggfls. unter Zwischenschaltung von regelbaren Pumpen erfolgen.

Die Rohrreaktoren sind so ausgelegt bzw. die Durchflußgeschwindigkeit ist so bemessen, daß mindestens ein überwiegender Teil der Hydrolyse, d.h. mindestens 60 %, vorzugsweise 85-95 % des Abbaus in den rohrförmigen temperierten Reaktoren stattfindet. Diese Spaltung findet also voneilhafterweise - wie dies nachstehend ausgeführt wird - als 1. Stufe in Form einer Rohspaltung statt. Die rohrförmigen Reaktoren enthalten vorzugsweise keinerlei Mischelemente, um eine einheitliche Vorwärtsbewegung der Suspension während der Hydrolyse ohne Vermischen zu gewährleisten. An den Verbindungsrohren können Probeentnahmestellen vorgesehen sein.

Das bereits zu einem überwiegenden Teil abgebaute Produkt, beispielsweise zu 90 %, kann sodann in eine oder mehrere weitere Reaktoren überführt werden, in denen der hydrolytische Abbau bis zum gewünschten Ausmaß (2. Stufe in Form einer Feinspaltung) durchgeführt wird. Bevorzugt wird der Rest der Hydrolyse in der Feinspaltungsstufe in Reaktoren durchgeführt, die statische Mischelemente aufweisen.

Mit Hilfe dieser Reaktoreinheiten ist es möglich, die Hydrolyse bis zu einem vorbestimmten Endwert zu führen und die Feinabstimmung des Verfahrens vorzunehmen. Diese Reaktoren sind deshalb vorteilhafterweise auch mit Mitteln zur Temperaturführung versehen. Auf diese Weise ist es möglich, den gewünschten Hydrolysegrad genau einzustellen. Der Hydrolysegrad wird vorzugsweise mit Hilfe von Viskositätsmessungen überprüft.

Erfindungsgemäß ist es bevorzugt, ein zweistufiges kontinuierliches Spaltungsverfahren, bestehend aus Roh- und Feinspaltung, durchzuführen. Andererseits kann jedoch auch ein einstufiges Spaltungsverfahren entsprechend der Rohspaltung durchgeführt werden, wenn der hydrolytische Abbau nicht notwendigerweise zu einem exakten vorbestimmten Abbaugrad führen muß, sondern innerhalb bestimmter Grenzen schwanken kann.

Die Strömung des flüssigen, zu hydrolysierenden Guts gegen die Schwerkraft hat den Vorteil, daß sich die in einem Reaktor befindlichen Schichten der Lösung/-Suspension praktisch nicht vermischen. Denn durch die Hydrolyse werden kontinuierlich Stärkespaltprodukte mit abnehmender Kettenlänge gebildet, was zur Folge hat, daß die Flüssigkeitsschichten eine immer geringere Viskosität, vom Boden des Reaktors nach oben betrachtet, aufweisen. In dem Reaktor befinden sich also Schichten mit einem stetig abnehmenden Viskositätsgefälle - von unten nach oben gegen die Schwerkraft betrachtet.

Die zu behandelnde Lösung wird bei der Hydrolyse mit einer solchen Geschwindigkeit durch den Reaktor bewegt, die im wesentlichen eine ungestörte Ausbildung dieses Viskositätsprofils gestattet. Nachdem die Reaktorlänge, also die Reaktionslänge bei dem kontinuierlichen Verfahren fixiert ist, legt die Fördergeschwindigkeit die gesamte Reaktionszeit fest, die in Abhängigkeit des gewählten Hydrolysegrads bestimmt wird.

Wird eine Mehrrohr-Reaktor-Anlage eingesetzt, so sind die Verbindungsrohre in ihrem Querschnitt so bemessen, daß die Schichten mit gleicher Viskosität von dem einen zum anderen Reaktor im wesentlichen ohne Störung, also ohne Vermischen der Schichten, überführt werden können, so daß in der nächsten Reaktoreinheit die Hydrolysebehandlung gegen die Schwerkraft erfolgen kann.

Man kann das Eigenschaftsprofil des erhaltenen Hydrolysats auch durch die Wahl der Konzentration der Anfangslösung oder Anfangssuspension sowie des Molekulargewichts der eingesetzten Stärke oder Stärkederivate, den Substitutionsgrad die Hydrolysetemperatur, die Säurekonzentration und dgl. beeinflussen.

Sollen modifizierte abgebaute Stärkeprodukte hergestellt werden, so wird vorzugsweise die Modifizierung der Stärke vor der Hydrolyse vorgenommen. Es ist bei dem erfindungsgemäßen Verfahren möglich und bevorzugt, sowohl das Modifizierungsverfahren als auch das Hydrolyseverfahren voll kontinuierlich durchzuführen.

So kann man Stärke, insbesondere abgebaute Stärke,. beispielsweise zur Ethoxylierung mit Ethylenoxid versetzen, wobei dem Gemisch soviel Natronlauge zugefügt wird, daß der gewünschte pH erreicht wird. Dieser pH liegt vorzugsweise im basischen Bereich und kann beispielsweise den Wert 13 haben.Das Mischen und die Reaktion können kontinuierlich durchgeführt werden, wobei die Umsetzung vorzugsweise in einem oder mebreren, hintereinander geschalteten, mit statischen Mischern versehenen Rohrreaktoren stattfindet. Das ethoxylierte Produkt wird sodann, wie vorstehend beschrieben, zwecks Durchführung der Hydrolyse mit Salzsäure versetzt und auf die gewünschte Temperatur gebracht und der Hydrolyse zugeführt.

Es war besonders überraschend, daß es mit Hilfe des erfindungsgemäßen Verfahrens möglich ist, kontinuierlich Stärke und/oder modifizierte Stärke hydrolytisch kontinuierlich mit einem einheitlichen Profil im Endprodukt abzubauen. Es war weiterhin besonders überraschend, daß man in den rohrförmigen Reaktoren ohne Durchmischung der Lösung mittels statischer Mischer oder bewegter Mischer ein Hydrolysat mit einer günstigen Molekulargewichtsverteilung erhält, insbesondere daß nicht durch Kanalbildung, wie zu befürchten war, ein sehr breites Molekulargewichtsspektrum entsteht.

Das erfindungsgemäße Verfahren bringt eine wesentliche Vereinfachung des Hydrolyseprozesses und führt somit zu erheblichen Kosteneinsparungen, da für Säurebydrolyseprozesse nur spezielle Stähle (beispielsweise HASTELLOY) eingesetzt werden können, was für Apparaturen, in denen statische Miscer vorhanden sind, sehr kostenaufwendig ist.

Mittels des erfindungsgemäßen Verfahrens ist es möglich, gezielt und reproduzierbar ein Hydrolysegut mit bestimmten Eigenschaften, herznstellen. Man kann so exakt definierte Endwerte einstellen wie Molekulargewicht, Molekulargewichtsverteilung, Substitutionsgrad. Diese Werte lassen sich reproduzierbar und auf längere Dauer konstant erzielen, während beim sogenannten batch-Betrieb die Schwankungen sehr groß sind und nur schwer zu steuern sind. Dies trifft nicht nur für den Abbau von Stärke zu, sondern auch für den Abbau von modifizierten Produkten, so daß es möglich ist, in Kombination mit einem Modifizierungsverfahren für die verschiedensten Einsatzzwecke Präparate mit den gewünschten Eigenschaften herzustellen.

Das Verfahren ist sehr flexibel und kann weitgehend automatisiert werden.

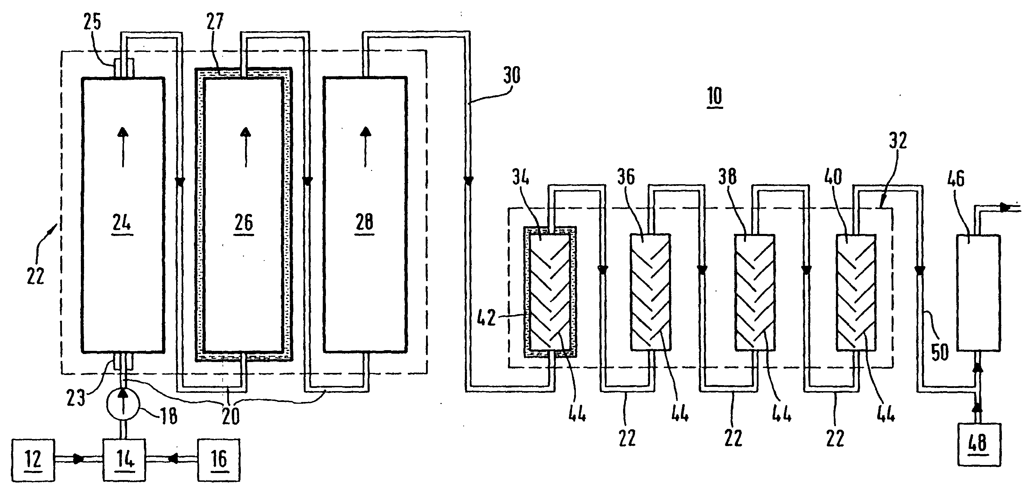

Das erfindungsgemäße Verfahren kann mit einer Anlage, wie sie schematisch in der Abbildung dargestellt ist, durchgeführt werden.

Mit 10 ist in der Zeichnung eine Vorrichtung zur kontinuierlichen Hydrolyse von Stärke oder Stärkederivaten angegeben. Von der Station 12 wird kontinuierlich zu hydrolysierende Hydroxyethylstärke-Lösung einem Mischer/Wärmetauscher 14 zugeführt, der mit einem Behälter 16 verbunden ist, der als Hydrolysierungsmittel, beispielsweise Salzsäure aufweist. In dem Mischer/Wärmetauscher 14 erfolgt eine pH-Wert-Einstellung auf 1-2 und eine Temperierung der Lösung auf eine vorgewählte Hydrolysetemperatur, beispielsweise 70-80°C.

Anschließend wird die Hydrolyselösung mittels einer Pumpe 18 mit einer vorgegebenen Pumprate durch eine erste Rohrleitung 20 einem Reaktor 22 (gemäß der Zeichnung drei Reaktoreinheiten 24-28) als Reaktionsstrecke (Haupt- oder Grobhydrolysestation) derart zugeführt, daß die Hydrolyselösung gegen die Schwerkraft von unten nach oben steigt, wie dies durch die Pfeilrichtung in den Reaktoreinheiten 24-28 dargestellt ist. Zu diesem Zwecke weist die Reaktoreinheit 24 in der Betriebslage einen unten angeordneten Einlaßstutzen 23 und einen oben angeordneten Auslaßstutzen 25 auf. Diese Stutzen 23 und 25 sind gleichermaßen bei den übrigen Reaktoreinheiten 26 und 28 vorgesehen. Die Rohrleitung 20 ist dabei in ihrem Querschnitt so dimensioniert, daß die jeweils aus dem Reaktor 24-28 ankommenden Hydrolyseschichten im wesentlichen nicht miteinander vermischt werden.

Andererseits kann es jedoch aber auch ausreichen, daß nur eine Reaktoreinheit 24 als Haupthydrolysestation (strichliert mit 22 dargestellt) eingesetzt wird, sofern diese ausreichend in seinem Volumen dimensioniert ist. Erfindungswesentlich ist es dabei, daß die langsam hochsteigende Hydrolyse-Lösung sich nicht in ihren Schichten miteinander vermischt, d.h. die einzelnen Schichten mit ihren unterschiedlichen Hydrolysezuständen sollen sich, ähnlich den Böden bei der fraktionierten Destillation, nicht miteinander vermischen. Infolgedessen kann am oberen Ausgangspunkt der Reaktoreinheiten 24-28 jeweils eine Lösung mit einem definierten Hydrolysegrad abgezogen werden, wobei anschließend eine Feineinstellung der Hydrolyse erfolgen kann. Die Reaktoreinheiten 24-28 werden daher als Haupthydrolysestation bezeichnet. Insbesondere sind die Reaktoreinheiten 24-28, in ihrem Innenraum nicht mit Mischelementen ausgerüstet, die eine Vermischung der gesamten Hydrolyse-Lösung bewirken könnten. Es soll also der Hydrolysegradient, der mit der Viskosität der einzelnen Schichten korreliert wird, im wesentlichen unverändert bleiben, d.h es soll keine Vermischung der einzelnen Schichten untereinander stattfinden.

Die Reaktoreinheiten 24-28 weisen vorteilhafterweise einen Temperiermantel 27 auf, wie er symbolisch bei der Reaktoreinheit 26 in der Zeichnung gezeigt ist. Dieser Temperiermantel ist mit einer auf einer vorbestimmten Temperatur gehaltenen Temperierflüssigkeit, üblicherweise Wasser, durchflossen und hält somit den Inhalt der jeweiligen Reaktoreinheiten 24-28 auf der gewünschten Temperatur. Letztere wird von Fall zu Fall dadurch bestimmt, daß an bestimmten Stellen die Reaktoreinheiten 24-28 Flüssigkeitsproben zur Bestimmung der jeweiligen Viskosität, d.h. des jeweiligen Hydrolysegrads entnommen werden und hieraus anhand von Tabellen und der bekannten Förderate der Endhydrolysegrad bestimmt wird.

Die Dimensionierung der Reaktoreinheiten 24-28 ist derart ausgelegt, daß eine Mindesdurchströmungsgeschwindigkeit bei einer vorbestimmten Viskosität sichergestellt ist, um eine Vermischung der jeweiligen Schichten infolge Diffusion zu verhindern. Letzere hängt u.a. im wesentlichen von der Viskosität ab. So liegt die Untergrenze der Strömungsgeschwindigkeit bei etwa 3 cm/min., wenn die Viskosität der zu hydrolysierenden Lösung bei etwa 20 mPa x s liegt. Vorteilhafterweise liegt die Strömungsgeschwindigkeit zwischen 5-20, insbesondere zwischen 10-15 cm/min.

Auch das Längen-/Durchmesserverhältnis der Reaktoreinheiten 24-28 wird vom Durchsatz der zu hydroliysierenden Lösung bestimmt. Vorteilhafterweise liegt dieses Verhältnis zwischen 10:1 bis 20:1.

Vorteilhafterweise ist die Haupthydrolysestation 22 durch eine zweite Rohrleitung 30 mit einer Feinhydrolysestation 32 (strichliert dargestellt) verbunden, wie sie durch die Reaktoreinheiten 34-40 gekennzeichnet ist. Diese Reaktoreinheiten 34-40 sind vorteilhafterweise mit einer Temperiereinheit 42 verbunden, beispielsweise durch einen Temperiermantel, durch den eine auf einer vorbestimmten Temperatur gehaltene Temperierflüssigkeit fließt und der dem Temperiermantel 27 entspricht. Hierdurch kann die für die Hydrolyse zweckmäßige Temperatur eingestellt werden, die vorteilhafterweise mittels der sich jeweils einstellenden Viskosität in der Hydrolyselösung überwacht und gesteuert wird. Des weiteren sind diese Reaktoreinheiten 34-40 mit schematisch dargestellten Mischelementen 44 ausgerüstet, so daß innerhalb der Reaktoren eine einheitliche Durchmischung stattfindet, um eine einheitliche Hydrolyse zu gewährleisten. Die Temperiereinheit 42 ist in der Zeichnung lediglich in Verbindung mit der Reaktoreinheit 34 dargestellt und kann natürlich auch bei den anderen Reaktoreinheiten 36-40 vorgesehen sein. Dergleichen wird sie aus einer üblichen nicht gezeigten Temperierflüssigkeitsquelle gespeist.

Es kann beispielsweise in der Haupthydrolysestation 22 eine Hydrolyse bis zu 95 % startfinden, während in der Feinhydrolysestation 32 nur noch 5 % des angestrebten Hydrolysegrads erzielt werden.

Wie vorstehend erwähnt, muß die Feinhydrolysestation 32 erfindungsgemäß nicht vorgesehen sein, da dort ein Hydrolyseprodukt mit einer engen Molekulargewichtsverteilung hergestellt wird. Ist eine solche enge Verteilung nicht notwendig, so kann eine solche Feinhydrolysestation 32 entfallen.

Wie in der Zeichnung dargestellt, wird die zu hydrolysierende Flüssigkeit in der Feinhydrolysestation 32 vorteilhafterweise ebenfalls von unten nach oben geführt, um Schwerkrafteinflüsse im wesentlichen auszuschalten.

Um die Hydrolyse zu stoppen, wird die Lösung am Ende der Hydrolyse in einer Neutralisationsstation 46 mit Natronlauge vermischt, die aus dem Vorratsbehälter 48 überführt wird. Insofern ist das Gefäß 46 mit einer dritten Rohrleitung 50 mit der Feinhydrolysestation 32 verbunden.

Anschließend wird das Gemisch zur weiteren Aufarbeitung, beispielsweise Diafiltration und Sprühtrocknung, aus dem Gefäß 46 in weitere, nicht gezeigte Bearbeitungsapparaturen überführt.

Die Erfindung wird durch folgendes Beispiel näher erläutert.

### Beispiel 1

Durch Zugabe von Wasser und Lauge wird eine 30%ige Stärkelösung hergestellt. Daraus wird in einer kontinuierlichen Stärkeethoxylierungsanlage mittels Ethylenoxid Hydroxylethylstärke mit einem Molekulargewicht von 1,4 Millionen Dalton hergestellt und mit einem Volumenstrom von ca. 11,3 l/h einem Rohrreaktor ohne Mischelemente zugeführt. Der Reaktor hat die Dimension von ca. 2,6m x DN 100. Die Ethoxylierung ist nicht Gegenstand der Erfindung, da auch eine Stärke für die nachfolgende Hydrolyse eingesetzt werden kann.

Der Hydroxyethylstärkelösung wird vor Einleiten in einen Hydrolysereaktor 0,2 l/h 25 %-ige HCl zudosiert; ferner wird die Reaktionslösung mittels eines Wärmeaustauschers auf eine Temperatur von 70°C gebracht, wobei der pH-Wert auf etwa 1,0 gebracht wird. Der Hydrolysereaktor wird mittels Temperierung auf 70°C gehalten, wobei es wesentlich ist, daß die unbehandelte Lösung den Reaktor, der ohne Mischelemente auskommt, von unten nach oben (gegen die Schwerkraft) durchströmt. Die Verweilzeit der Lösung beträgt ca. 2 Stunden. Dabei wurde das Molekulargewicht von 1,4 Mio. auf 300 000 Dalton reduziert.

In einem vorteilhafterweise nachgeschalteten Feinhydrolyseteil, der aus mehreren Reaktoren mit statischen Mischelementen besteht, wird ein Endmolekulargewicht (Gewichtsmittel) von etwa 250.000 Dalton eingestellt. Die Hydrolyse wird sodann mittels Neutralisation beendet. Das Gemisch wird mittels Diafiltration mit Hilfe einer Membran einer Ausschlußgrenze von 50.000 Dalton gereinigt. Das getrocknete Produkt ist hervorragend geeignet als Plasmastreckmittel.

## Patentansprüche

1. Verfahren zur kontinuierlichen Herstellung von hydrolytisch abgebauten Stärkederivaten, die gegebenenfalls substituiert sind, durch Umsetzen mit einem Säurehydrolysemittel in einem wäßrigen Medium und anschließendes Neutralisieren zum Abbruch der Hydrolyse, **dadurch gekennzeichnet, daß** man kontinuierlich eine Lösung oder Suspension, die die zu hydrolysierende, gegebenenfalls substituierte Stärke enthält, durch eine Reaktionsstrecke im wesentlichen vermischungsfrei gegen die Schwerkraft in der Hydrolysestufe führt.

2. Verfahren nach Anspruch 1, **dadurch gekennzeichnet, daß** die Reaktions-strecke zumindest einen rohrförmigen Reaktor (24-28) aufweist, der in der Betriebslage einen unten angeordneten Einlaßstutzen (23) und einen oben angeordneten Auslaßstutzen (25) aufweist.

3. Verfahren nach Anspruch 1 oder 2, **dadurch gekennzeichnet, daß** man im Anschluß an die Haupthydrolyse gemäß Anspruch 1 eine Feinhydrolyse durchführt, bei der man die grob hydrolysierte Stärkelösung einem rohrförmigen Reaktor (34) mit Mischelementen (44) bei einer vorbestimmten Temperatur zuführt.

4. Verfahren nach einem der Ansprüche 1 bis 3, **dadurch gekennzeichnet, daß** die rohrförmigen Reaktoren (24-28 und 34-40) im Betriebszustand im wesentlichen senkrecht angeordnet werden und das zu hydrolysierende Produkt von unten nach oben geführt wird.

5. Verfahren nach einem der Ansprüche 1 bis 4, **dadurch gekennzeichnet, daß** die rohrförmigen Reaktoren auf eine vorbestimmte Temperatur von 25-100°C temperiert werden.

6. Verfahren nach einem oder mehreren der Ansprüche 1 bis 5, **dadurch gekennzeichnet, daß** man die Haupthydrolyse im rohrförmigen temperierten Reaktor (22) zu 60-95 % durchführt.

7. Verfahren nach einem oder mehreren der Ansprüche 1 bis 6, **dadurch gekennzeichnet, daß** man veretherte Stärke einsetzt.

8. Verfahren nach Anspruch 3, **dadurch gekennzeichnet, daß** man die Feinhydrolyse mit mehreren, mit statischen Mischelementen (44) versehenen Reaktoren (34-40) durchführt.

9. Verfahren nach einem oder mehreren der Ansprüche 1 bis 8, **dadurch gekennzeichnet, daß** man teilabgebaute Stärke kontinuierlich mit Ethylenoxid in basischem Milieu ethoxyliert, das ethoxylierte Produkt mit Mineralsäure ansäuert, die Haupthydrolyse bei einer Reaktionstemperatur von 60-100°C durchführt und die Hydrolyse durch Neutralisation mit Lauge und Abkühlen beendet

10. Verwendung des nach einem der Ansprüche 1 bis 9 hergestellten hydrolytisch abgebauten Produkts als Plasmastreckmittel oder zur Herstellung von Dialyselösungen.

11. Vorrichtung zur Durchführung des Verfahrens nach Anspruch 1 mit einer Einspeiseeinrichtung (12) für Stärkelösung,
einem Behälter (16) für ein Hydrolysierungsmittel,
einer Misch- und Erwärmungsstation (14) zum Mischen der Stärkelösung mit dem Hydrolysierungsmittel und Erwärmen des Gemischs auf eine vorbestimmte Temperatur,
einer Pumpanordnung (18) zum Einspeisen des Gemischs in wenigstens einen Reaktor (22),
einer Rohrleitung (20), die sämtliche Einheiten miteinander verbindet, sowie einer Neutralisationsstation (46) zum Neutralisieren des Gemischs,
wobei der Reaktor (22) in der Gebrauchslage im wesentlichen senkrecht angeordnet ist und einen untenliegenden Einlaßstutzen (23) und einen obenliegenden Auslaßstutzen (25) aufweist und die Pumpanordnung (18) so betrieben ist, daß sie kontinuierlich mit einer vorbestimmten Pumprate die Stärkelösung dem untenliegenden Einlaßstutzen (23) zuführt, so daß die Stärkelösung gegen die Schwerkraft durch den Reaktor (22) zum Auslaßstutzen (25) gefördert wird.

12. Vorrichtung nach Anspruch 11, **dadurch gekennzeichnet, daß** dem Reaktor (22) als Haupthydrolysestation eine Feinhydrolysestation (32) in Form wenigstens einer Reaktoreinheit (34-40) nachgeschaltet ist, die jeweils Mischelemente (44) aufweist.

13. Vorrichtung nach Anspruch 11 oder 12, dadurch gekermzeichnet, daß die Reaktoren (24-28 und 34-40) jeweils mit einer Temperiereinheit (27, 42) versehen sind.

14. Verfahren nach Anspruch 7, **dadurch gekennzeichnet, daß** man veretherte Wachsmaisstärke einsetzt.

15. Verfahren nach Anspruch 7, **dadurch gekennzeichnet, daß** man mit Ethylenoxid und/oder Propylenoxid veretherte Stärke einsetzt

16. Verfahren nach Anspruch 14, **dadurch gekennzeichnet, daß** man mit Ethylenoxid und/oder Propylenoxid veretherte Wachsmaisstärke einsetzt.

## Claims

1. A method for the continuous production of hydrolytically degraded starch derivatives which are possibly substituted, by reaction with an acid hydrolysing medium in an aqueous medium followed by neutralisation to breakdown the hydrolysis, **characterised in that** a solution or suspension which contains the possibly substituted starch to be hydrolysed is passed continuously through a reaction path substantially mixture free against the force of gravity in the hydrolysis stage.

2. A method according to claim 1, **characterised in that** the reaction path comprises at least one tubular reactor (24 - 28) which, in the operating position, comprises an inlet nozzle (23)disposed at the bottom and an outlet nozzle (25) disposed at the top.

3. A method according to claim 1 or 2, **characterised in that** following the main hydrolysis according to claim 1, a fine hydrolysis is carried out in which the coarsely hydrolysed starch solution is fed to a tubular reactor (34) with mixing elements (44) at a predetermined temperature.

4. A method according to one of claims 1 to 3, **characterised in that** the tubular reactors (24 - 28 and 34 - 40) are, in the operating state, substantially vertical and **in that** the product to be hydrolysed is fed upwards from below.

5. A method according to one of claims 1 to 4, **characterised in that** the tubular reactors are maintained at a predetermined temperature of 25 to 100°C.

6. A method according to one or more of claims 1 to 5, **characterised in that** the main hydrolysis is carried out in the tubular temperature controlled reactor (22) at 60 to 95%.

7. A method according to one or more of claims 1 to 6, **characterised in that** esterified starch is used.

8. A method according to claim 3, **characterised in that** the fine hydrolysis is carried out with a plurality of reactors (34 - 40) provided with static mixing elements (44).

9. A method according to one or more of claims I to 8, **characterised in that** partially degraded starch is continuously ethoxylated with ethylene oxide in a basic medium, the ethoxylated product is acidulated with mineral acid, the main hydrolysis is carried out at a reaction temperature of 60 to 100°C and hydrolysis is completed by neutralisation with lye and cooling.

10. Use of the hydrolytically degraded product produced in accordance with one of claims 1 to 9 as a plasma diluent or for the production of dialysis solutions.

11. An apparatus for carrying out the method according to claim 1, with a feed means (12) for starch solution, a container (16) for a hydrolysing agent, a mixing and heating station (14) for mixing the starch solution with the hydrolysing medium and heating the mixture to a predetermined temperature, a pump arrangement (18) for feeding the mixture into at least one reactor (22), a pipeline (20) which combines all the units with one another, and a neutralisation (46) for neutralising the mixture, whereby the reactor (22), in the position of use, is arranged substantially vertically and has at the bottom an inlet connector (23) and at the top an outlet connector (25), the pump arrangement (18) being so operated that it continuously and at a predetermined pumping rate, feeds the starch solution to the inlet connector (23) which is at the bottom, so that against the force of gravity, the starch solution is conveyed through the reactor (22) to the outlet connector (25).

12. An apparatus according to claim 11, **characterised in that** on the downstream side of the reactor (22) as the main hydrolysing station there is a fine hydrolysing station in the form of at least one reactor unit (34 - 40) which comprises mixing elements (44).

13. An apparatus according to claim 1 or 12, **characterised in that** the reactors (24 - 28 and 34 - 40) are each connected to an air conditioning unit (27, 42).

14. A method according to claim 7, **characterised in that** etherified wax maize starch is used.

15. A method according to claim 7, **characterised in that** starch etherified with ethylene oxide and/or propylene oxide is used.

16. A method according to claim 14, **characterised in that** wax maize starch etherified with ethylene oxide and/or propylene oxide is used.

## Revendications

1. Procédé pour la préparation en continu de dérivés d'amidon soumis à une décomposition par hydrolyse, qui sont le cas échéant substitués, par mise en réaction avec un agent d'hydrolyse acide dans un milieu aqueux et par neutralisation ultérieure pour mettre un terme à l'hydrolyse, **caractérisé en ce qu'**on guide en continu une solution ou une suspension, qui contient l'amidon à hydrolyser, le cas échéant substitué, à travers un tronçon de réaction, essentiellement en l'absence de mélangeage, à l'encontre de la force de pesanteur, dans l'étape d'hydrolyse.

2. Procédé selon la revendication 1, **caractérisé en ce que** le tronçon de réaction présente au moins un réacteur tubulaire (24 - 28) qui présente, en état de marche, une tubulure d'entrée (23) disposée dans la partie inférieure et une tubulure de sortie (25) disposée dans la partie supérieure.

3. Procédé selon la revendication 1 ou 2, **caractérisé en ce qu'**on procède, directement après l'hydrolyse principale selon la revendication 1, à une hydrolyse fine dans laquelle on achemine, à une température prédéterminée, la solution d'amidon soumise à une hydrolyse grossière, à un réacteur tubulaire (34) équipé d'éléments de mélange (44).

4. Procédé selon l'une quelconque des revendications 1 à 3, **caractérisé en ce qu'**on dispose les réacteurs tubulaires (24 - 28 et 34 - 40) en état de marche essentiellement à la verticale et on guide le produit à hydrolyser de bas en haut.

5. Procédé selon l'une quelconque des revendications 1 à 4, **caractérisé en ce qu'**on maintient les réacteurs tubulaires à une température prédéterminée de 25 à 100 °C.

6. Procédé selon l'une quelconque des revendications 1 à 5, **caractérisé en ce qu'**on effectue l'hydrolyse principale jusqu'à concurrence de 60 à 95 % dans un réacteur tubulaire (22) maintenu à une température constante.

7. Procédé selon l'une quelconque des revendications 1 à 6, **caractérisé en ce qu'**on met en oeuvre de l'amidon éthérifié.

8. Procédé selon la revendication 3, **caractérisé en ce qu'**on effectue l'hydrolyse fine avec plusieurs réacteurs (34 - 40) équipé d'éléments de mélange statiques (44).

9. Procédé selon l'une quelconque des revendications 1 à 8, **caractérisé en ce qu'**on soumet de l'amidon, soumis à une décomposition partielle, à une éthoxylation dans un milieu basique avec de l'oxyde d'éthylène en continu, on acidifie le produit éthoxylé avec un acide minéral, on effectue l'hydrolyse principale à une température réactionnelle de 60 à 100 °C et on met un terme à l'hydrolyse par neutralisation avec de la lessive alcaline et par refroidissement.

10. Utilisation du produit soumis à une décomposition hydrolytique préparé conformément à l'une quelconque des revendications 1 à 9, à titre de diluant du plasma ou pour la préparation de solutions de dialyse.

11. Dispositif pour la mise en oeuvre du procédé selon la revendication 1, comprenant un dispositif d'alimentation (12) pour une solution d'amidon, un réservoir (16) pour un agent d'hydrolyse, un poste de mélange et de réchauffement (14) pour le mélange la solution d'amidon avec l'agent d'hydrolyse et pour le réchauffement du mélange à une température prédéterminée, un agencement de pompage (18) pour alimenter le mélange dans au moins un réacteur (22), un conduit tubulaire (20) qui relie l'ensemble des unités les unes aux autres, ainsi qu'un poste de neutralisation (46) pour la neutralisation du mélange, dans lequel le réacteur (22) est disposé, lorsqu'on l'utilise, essentiellement à la verticale et présente une tubulure d'entrée inférieure (23) et une tubulure de sortie supérieure (25), l'agencement de pompage (18) étant mis en service de telle sorte qu'il achemine en continu, avec un débit de pompage prédéfini, la solution d'amidon à la tubulure d'entrée inférieure (23) de telle sorte que la solution d'amidon est transportée à l'encontre de la force de pesanteur à travers le réacteur (22) jusqu'à la tubulure de sortie (25).

12. Dispositif selon la revendication 11, **caractérisé en ce qu'**un poste d'hydrolyse fine (32) sous la forme d'au moins une unité de réacteur (34 - 40) est monté en aval du réacteur (22) faisant office de poste d'hydrolyse principale, le premier cité présentant respectivement des éléments de mélange (44).

13. Dispositif selon la revendication 11 ou 12, **caractérisé en ce que** les réacteurs (24 - 28 et 34 - 40) sont respectivement équipés d'une unité (27, 42) de maintien de la température constante.

14. Procédé selon la revendication 7, **caractérisé en ce qu'**on met en oeuvre de l'amidon de maïs glutineux éthérifié.

15. Procédé selon la revendication 7, **caractérisé en ce qu'**on met en oeuvre de l'amidon éthérifié avec de l'oxyde d'éthylène et/ou de l'oxyde de propylène.

16. Procédé selon la revendication 14, **caractérisé en ce qu'**on met en oeuvre l'amidon de maïs glutineux éthérifié avec de l'oxyde d'éthylène et/ou de l'oxyde de propylène.
